(19) Europäisches Patentamt

European Patent Office

Office européen des brevets

(11) **EP 1 087 749 B1**

(12) **EUROPÄISCHE PATENTSCHRIFT**

(45) Veröffentlichungstag und Bekanntmachung des Hinweises auf die Patenterteilung:
**12.11.2003  Patentblatt 2003/46**

(51) Int Cl.[7]: **A61K 7/48**

(21) Anmeldenummer: **99941385.9**

(86) Internationale Anmeldenummer:
**PCT/DE99/01851**

(22) Anmeldetag: **22.06.1999**

(87) Internationale Veröffentlichungsnummer:
**WO 99/066881 (29.12.1999 Gazette 1999/52)**

(54) **KOSMETISCHE WIRKSTOFFZUBEREITUNG MIT HOHEM RADIKALSCHUTZFAKTOR**

COSMETIC PREPARATION OF ACTIVE SUBSTANCES WITH HIGH PROTECTION FACTOR AGAINST FREE RADICALS

PREPARATION COSMETIQUE DE PRINCIPES ACTIFS A FACTEUR ELEVE DE PROTECTION CONTRE LES RADICAUX LIBRES

(84) Benannte Vertragsstaaten:
**DE ES FR GB IT MC NL**

(30) Priorität: **24.06.1998  DE 19830004**
**23.12.1998  DE 19860754**

(43) Veröffentlichungstag der Anmeldung:
**04.04.2001  Patentblatt 2001/14**

(73) Patentinhaber: **Coty B.V.**
**2031 CC Haarlem (NL)**

(72) Erfinder:
• **GOLZ-BERNER, Karin**
**MC-98000 Monaco (MC)**
• **ZASTROW, Leonhard**
**MC-98000 Monaco (MC)**

(74) Vertreter: **Walter, Wolf-Jürgen et al**
**Gulde Hengelhaupt Ziebig & Schneider**
**Schützenstrasse 15-17**
**10117 Berlin (DE)**

(56) Entgegenhaltungen:
EP-A- 0 518 192        EP-A- 0 768 079
WO-A-93/24106          WO-A-96/06532
FR-A- 2 750 331        US-A- 4 264 592
US-A- 4 402 856        US-A- 4 505 902
US-A- 5 306 486        US-A- 5 614 489

**Beschreibung**

Die erfindungsgemäße kosmetische Wirkstoffzubereitung schützt in besonders wirksamer Weise gegen den Angriff freier Radikale auf die Haut sowohl allein als auch in Kombination mit anderen Wirkstoffen.

Bekanntlich sind freie Radikale, wie Superoxid-Ionen, Hydroxy-Radikale, Oxide) für einen Gewebeabbau und damit für die Erscheinung des Alterns der Haut verantwortlich. Die Proteine und Lipide der Zellmembranen werden zerstört, die DNA beschädigt und eine Schlüsselsubstanz der Haut, Hyaluronsäure, wird ebenfalls abgebaut. Unter normalen biologischen Bedingungen gibt es ein Gleichgewichtsverhältnis zwischen auftretenden freien Radikalen und deren Eindämmung durch körpereigene chemische oder enzymatische Systeme. Durch zusätzliche Streßfaktoren von außen, wie agressive Atmosphäre, Tabakrauch, Ultraviolettstrahlung usw. werden diese körpereigenen Abwehrsysteme überfordert und das Gleichgewicht zugunsten der freien Radikale gestört. Es treten Entzündungs- oder Alterungserscheinungen der Haut auf, und ein Ausgleich durch kosmetische Produkte ist angezeigt.

Es sind bereits eine Reihe von Produkten für diesen Zweck vorgeschlagen worden, die oftmals Vitaminmischungen mit den Vitaminen A, C und E enthalten oder Zusätze von Superoxiddismutase oder Extrakte aus bestimmten pflanzlichen oder tierischen Lebewesen. So ist aus der US-A-5,629,185 eine kosmetische Zusammensetzung bekannt, die Ultraschallaufschlußprodukte von Hefen oder anderen Zelldispersionen enthält. Aus der WO96/29048 ist ein Kosmetikum bekannt, das kondensierte Abbauprodukte pflanzlicher oder tierischer Herkunft enthält. Weiterhin gibt es eine Vielzahl von Veröffentlichungen, in denen die Verwendung reiner Pflanzenextrakte für kosmetische Zwecke beschrieben ist, wie z.B. in der WO97/45100 mit einem Gemisch von sieben verschieden Extrakten zum Bekämpfung von Cellulite.

US-A-4,402,856 offenbart Mikrokapseln mit definierter Freisetzungstemperatur, die Tannine enthalten, beispielsweise Gallussäure. Die WO 93/24106 beschreibt eine kosmetische Zusammensetzung, die ein Proanthocyanidin-Oligomeres in Liposomen enthält. EP-A-0768079 beschreibt ein Haarwachstumsmittel mit Proanthocyanidinen. WO 96/06532 offenbart eine Kombination von Protein oder Peptid mit einem zellwandabbauendem Enzym. Die US-A-5,614,489 betrifft eine kosmetische Zusammensetzung mit einem Collagenase/Elastase-Inhibitor. Die EP-A-0518192 betrifft ein naturkosmetisches Mittel, das u.a. Zitronensaft und Kiwi-Saft enthält. Die FR-A-2750331 beschreibt kosmetische Zusammensetzungen mit Anteilen von nicht-peroxidischen ölen und gegebenenfalls Mandelöl. Die US-A-4,505,902 offenbart eine kosmetische Zusammensetzung, die u.a. Aprikosenkernöl enthält. US-A-4,264,592 betrifft auf Zitrusfrüchten basierende Kosmetika. US-A-5,306,486 offenbart kosmetische Sonnenschutzzubereitungen, die u.a. Blattextrakte von grünem Tee enthalten.

Die Suche nach weiteren wirksamen Stoffen ist ein wesentliches Element kosmetischer Forschung. Weiterhin problematisch bei vielen dieser Produkte ist es, daß die gegen freie Radikale wirksamen Stoffe ihr Fängerpotential innerhalb der fertigen kosmetischen Zusammensetzung oftmals nicht auf Dauer beibehalten, d.h. es sind besondere Formulierungen erforderlich, damit die Wirksamkeit der Radikalfänger auf Dauer erhalten bleibt.

Zum anderen scheint sich in der kosmetischen Industrie die Kenntnis bisher noch nicht durchgesetzt zu haben, daß die Möglichkeit besteht, das antioxidative Potential der Haut zu messen (DE 4328639) und neuerdings auch mit Hilfe eines relativ einfachen Verfahrens den Radikalschutzfaktor einer kosmetischen Zubereitung zu bestimmen und damit gezielt Materialien in eine solche Zubereitung einzubeziehen.

Es ist Aufgabe der vorliegenden Erfindung, eine kosmetische Wirkstoffzubereitung bereitzustellen, die ein besonders hohes Radikalschutzpotential hat.

Eine weitere Aufgabe der Erfindung ist die Bereitstellung einer Wirkstoffzubereitung, die ihr Radikalschutzpotential über eine langen Zeitraum hält.

Eine weitere Aufgabe der Erfindung ist die Bereitstellung spezieller kosmetischer Zusammensetzungen, die diese Wirkstoffzubereitung enthalten und speziell solcher Wirkstoffzubereitungen, die eine weitere Verbesserung der Eigenschaften insbesondere hinsichtlich der Öffnung der Hautporen erreicht.

Erfindungsgemäß ist die kosmetische Wirkstoffzubereitung mit hohem Radikalschutzfaktor gekennzeichnet durch einen Gehalt an

(a) einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew- % Gallussäure enthält, wobei der Gehalt von (a), das in einer an Mikrokapseln gebundenen Wirkstoffkonzentration von 2 Gew- % vorliegt, im Bereich von 0,1 bis 10 Gew- % liegt;

(b) einem durch Extraktion gewonnenen Seidenraupenextrakt, der das Peptid Cecropine, Aminosäuren und ein Vitamingemisch enthält, wobei der Gehalt von (b) im Bereich von 0,1 bis 10 Gew-% liegt;

(c) einem nichtionischen, kationischen oder anionischen Hydro-Gel oder Gemisch von Hydro-Gelen mit einem Gehalt von (c) im Bereich von 0,1 bis 5 Gew-%;

(d) einem oder mehreren Phospholipiden mit einem Anteil von 0,1 bis 30 Gew-%;

(e) Wasser bis 100 Gew-%,

jeweils bezogen auf die Gesamtmasse der Wirkstoffzubereitung.

Gegebenenfalls kann die Wirkstoffzubereitung weiterhin enthalten

(f) ein wenigstens 150 Einheiten Superoxiddismutase pro ml enthaltendes Ultraschall-Aufschlußprodukt einer Hefe, wobei der Gehalt des Aufschlußproduktes (f) im Bereich von 0 bis 4 Gew-% liegt;

(g) ein Extrakt von Acerolafrüchten Malpighia punidifolia, wobei der Gehalt (g) im Bereich von 0-20 Gew-% liegt; und

(h) ein Gemisch aus 0,1 Gew-% Micrococcus luteus-Extrakt, Retinylpalmitat und Tocopherylacetat in mit Phospholipiden zubereiteter liposomaler Form, sowie freies Retinylpalmitat; jeweils bezogen auf das Gesamtgewicht der Wirkstoffzubereitung.

Für eine Ausführungsform der Erfindung mit der Wirkkomponente (h) sind die Bestandteile der Zubereitung, bezogen auf das Gesamtgewicht eines kosmetischen Präparates, wie folgt vorhanden:

Wirkstoffkapseln gemäß (a) im Bereich von 0,1 bis 10 Gew-%,

Hydro-Gel gemäß (b) im Bereich von 0,1 bis 5 Gew-%,

gekapseltes Retinylpalmitat gemäß (h) 0,001 bis 5 Gew-%,

gekapseltes Tocopherylacetat gemäß (h) 0,001 bis 2 Gew-% ,

freies Retinylpalmitat gemäß (h) 0,1 bis 5 Gew-%,

Phospholipide 0,2 bis 5 Gew-%,

Wasser als restlicher Bestandteil bis 100 Gew-% und/oder andere Hilfs- und Trägerstoffe.

Der erfindungsgemäße Quebracho-Rindenextrakt bzw. dessen Hydrolyseprodukt enthält einen sehr hohen Anteil Proanthocyanidine, die kondensierte Tannine darstellen. Diese in Form von Oligomeren auftretenden Verbindungen sowie der geringe Anteil an Gallussäure zeigt in dieser Kombination und in Konzentrationen zwischen 1 und 10 Gew-% eine deutliche Radikalschutzwirkung, die die Wirkung von Superoxiddismutase (SOD) bei weitem übertrifft. Die Aktivität gegen freie Radikale wurde mit der von SOD verglichen und lag bei einer 1 Gew-%igen Lösung des Extraktes bei 42 % (SOD 4 %), einer 2,5 Gew-%igen Lösung bei 83 % (SOD 15 %) und einer 5 Gew-%igen Lösung bei 100 % (SOD 38 %).

Bevorzugt enthält der Extrakt (a) wenigstens 95 Gew- % Proanthocyanidin-Oligomere und höchstens 5 Gew-% Gallussäure, insbesondere wenigstens 99 Gew- % Proanthocyanidin-Oligomere und höchstens 1 Gew-% Gallussäure.

Der Gehalt von (a) beträgt 1 bis 10 Gew-%, wobei der Wirkstoff aus der Quebrachorinde in Mikrokapseln eingeschlossen ist. Die Mikrokapseln bestehen z.

B. aus Petrolatum, Natriumtristearat, Agar, Phenonip und Wasser.

Der Seidenraupenextrakt (b) wird durch Extraktion der Seidenraupe (Bombyx mori) mit Propylenglycol gewonnen und enthält Vitamine, Aminosäuren und das Peptid Cecropine, das eine besondere antibakterielle Wirksamkeit aufweist. In den letzten Jahren haben eine Reihe von Untersuchungen an der Hämolymphe und der Kutikularmatrix des Seidenwurmes gezeigt, daß nicht nur antibakterielle Peptide sondern auch Inhibierungsmittel von insbesondere fungalen Proteasen darin enthalten sind. Weiterhin zeigen derartige Extrakte sauerstoffverbrauchende Eigenschaften, wodurch sie den Zellmetabolismus aktivieren, und sie haben feuchtigkeitshaltende Eigenschaften, eine deutliche Heilwirkung auf Hautläsionen durch Verkürzung der Heilungszeit sowie eine glättende Wirkung auf die Haut.

Bevorzugt enthält der Extrakt (b) die Aminosäuren Aspertinsäure, Asparagin, Threonin, Serin, Glutaminsäure, Prolin, Glycin, Alanin, Valin, Cystein, Methionin, Isoleucin, Leucin, Tyrosin, Phenylalanin, Lysin, Histidin, Arginin.

Weiterhin bevorzugt enthält der Extrakt (b) ein Vitamingemisch mit den Vitaminen $B_1$, $B_2$, $B_5$, $B_6$, $B_8$, $B_9$, $B_{12}$, PP, A, E und C.

Die Bestandteile (a) und (b) liegen vorzugsweise in Konzentrationen von jeweils 0,1 bis 3 Gew-% in der Wirkstoffzubereitung vor, insbesondere 0,5 bis 3 Gew-%.

Das erfindungsgemäß enthaltende Gel, das auch ein Gemisch verschiedener Gele sein kann, ist ein Hydrogel, das wasserlöslich ist bei Temperaturen von höher als etwa 40 bis 50 °C und die Gelstruktur bei niedrigeren Temperaturen zwischen 10 und 30 °C ausbildet. Beispiele für derartige Gele sind nichtionische Polymere wie Polyvinylakohol, Polyvinylpyrrolidonmodifizierte Maisstärke und Hydroxyethylcellulose; kationische Polymere wie kationischer Guar, kationische Cellulose, synthetische kationische Polymere; oder Gele wie Gelatine, Carrageenan, Bentonit-Gele, Copolymer-Gele wie Carbomer.

Die erfindungsgemäß enthaltenen Phospholipide sind ausgewählt unter Phosphatidylcholin, Phosphatidylethanolamin, Phosphatidylinositol, Phosphatidylserin, Phosphatidsäure und Lysolecithine sowie Gemischen davon. Bekannte Produkte sind beispielsweise Phoslipon®. Der Anteil der Phospholipide liegt im Bereich von 0,1 bis 30 Gew-%, vorzugsweise 0,5 bis 20 Gew-%.

Die Komponenten (a) und (b) der Wirkstoffzubereitung bilden mit den Phospholipiden (d) vermutlich assoziationsähnliche Gebilde verschiedener Moleküle, die wiederum in der sich ausbildenden Struktur des Gels (c)+(e) in weitgehend gleichmäßiger Verteilung angelagert werden und die hier insgesamt als "Assoziationskomplex" bezeichnet werden. Darin integriert können auch Anteile des SOD-haltigen Hefeaufschlußproduktes und des Acerola-Extraktes sowie bestimmter Pflanzenextrakte sein.

Das gekapselte Gemisch aus 0,1 Gew-% Micrococcus luteus-Extrakt, Retinylpalmitat und Tocopherylacetat liegt in mit Phospholipiden zubereiteter liposomaler Form vor, wobei der Gehalt an Retinylpalmitat und Tocopherylacetat vorteilhaft im Bereich von 0,001 bis 1 Gew-% betragen kann. Der Anteil der Phospholipide in diesem gekapselten Gemisch liegt allgemein zwischen 5 und 40 Gew-%.

Die Wirkstoffzubereitung in Form des Assoziationskomplexes kann zusätzlich ein Gemisch der Vitamine A, B und C sowie zusätzlich SOD und/oder Extrakte von Acerolafrüchten enthalten. Es können jedoch auch Vitamine sowie andere Antioxidationsmittel in der Gesamtzusammensetzung der kosmetischen Zubereitung enthalten sein.

Die Wirkstoffzubereitung kann erfindungsgemäß neben den Grundbestandteilen (a) bis (e) weiterhin verschiedene Pflanzenextrakte enthalten, wie Citrusschalen- oder -blatt-Extrakte (Citrus bigaradia, Citrus hystrix, Citrus aurantifolia, Citrofurtunella microcarpa, Citrus aurantium, Citrus reticulata), Bitterorange-Extrakt (Schale oder Frucht), Kirsch-Extrakt der spanischen Cherry-Kirsche, Kiwi-Extrakt (Actinidia chinensis), Papayafrucht- Extrakt (Caricae papayae), Tee-Extrakt [Blätter von grünem oder schwarzem Tee, Blätter oder Rinde von New Jersey Tee (Ceanthus velutinas)], Kaffebohnen-Extrakt (INCI-Name: Coffee Bean Extract; von grünen oder gerösteten Bohnen), Prunus-Extrakt (Prunus armeniaca, Prunus dulcis, Prunus persica, Prunus domestica, Prunus spinosa, Prunus serotina, Prunus virginiana), Extrakte der Rinde des mexikanischen Hautbaumes (Mimosa tenuiflora), Angelikawurzel-Extrakt (Angelica archangelica). Derartige Pflanzenextrakte sind kommerziell erhältlich, z.B. von DRAGOCO, Holzminden, Deutschland.

Der Gehalt an diesen Pflanzenextrakten kann zwischen 0 und 40 Gew-% liegen vorzugsweise 0,1 bis 40 Gew-%, insbesondere 0,5 bis 20 Gew-%, wobei auch Gemische dieser Extrakte sowie Gemische mit den Bestandteilen (f) und (g) der Wirkstoffzubereitung enthalten sein können.

Der Zusatz der oben genannten Pflanzenextrakte kann den Radikalschutzfaktor je nach Pflanze und entsprechend zugesetzter Menge um ein Mehrfaches erhöhen, wobei vermutlich synergistische Wechselwirkungen auftreten, deren Zusammenhänge noch nicht vollständig geklärt werden konnten.

Zu den in der Erfindung einsetzbaren Antioxidationsmitteln gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B $\alpha$-Carotin, $\beta$-Carotin; Harnsäure und Derivate davon; $\alpha$-Hydroxysäuren wie Citronensäure, Milchsäure, Äpfelsäure; Stilbene und deren Derivat usw.

Vitamine können auch im Gemisch mit Enzymen als weiterer Anteil in der Wirkstoffzubereitung oder in der kosmetischen Zusammensetzung neben der Wirkstoffzubereitung enthalten sein. Der Gehalt kann wenigstens 0,5 Gew-% eines Gemisches aus Enzymen und Vitaminen betragen, das wenigstens 150 Einheiten/ ml (U/ml) Superoxiddismutase (SOD) enthält.

Das eingesetzte Gemisch aus Enzymen und Vitaminen ist vorzugsweise ein durch Ultraschallbehandlung hergestelltes Aufschlußprodukt einer Hefe, wobei das Aufschlußprodukt SOD, Protease, Vitamin $B_2$, Vitamin $B_6$, Vitamin $B_{12}$, Vitamin $D_2$ und Vitamin E enthält. Vorzugsweise enthält es wenigstens 150 U/ml SOD, Protease und die Vitamine B und D, wobei das Verhältnis SOD:Protease als internationale Einheiten wenigstens im Bereich von 3:1 bis 8:1 liegt.

Besonders vorteilhaft für die Herstellung des Enzym-/Vitamingemisches ist ein Aufschlußverfahren mittels Ultraschall, das in der DE 4241154C1 beschrieben ist und bei dem in einer Ultraschall-Durchflußzelle eine Zelldispersion oder Suspension durch einen Beschallungsraum geführt wird, bei dem die Sonotrode zur Hälfte bis Zweidrittel ihrer Länge in die Durchflußzelle hineinragt und in das zu beschallende Medium eintaucht. Dabei hat die Sonotrode einen Winkel von 80,5 bis 88,5°, und das Verhältnis Eintauchlänge der Sonotrode in mm zum Beschallungsvolumen in ml wird auf einen Wert von 1:1,1 bis 1:20 eingestellt. Der Feststoffanteil in dem zu beschallenden Medium liegt im Bereich von 1:0,02 bis 1:2,2 (Gew.-%).

Als Zelldispersion können Hefen, wie Bäckerhefe, Brauereihefe, Weinhefe sowie besonders behandelte Hefen, wie z.B. SOD-angereicherte Hefen, eingesetzt werden. Eine vorteilhaft einzusetzende Zelldispersion enthält z. B. Saccharomyces cerevisiae.

Der Zusatz von z.B. 1 Gew-% eines solchen Hefeaufschlußproduktes aus Bäckerhefe als wahlweisen Bestandteil des Assoziationskomplexes kann den an sich schon hohen Radikalschutzfaktor von z.B. 1620 um etwa das 2-fache erhöhen auf 3150. Zum Radikalschutzfaktor werden weiter unten noch nähere Ausführungen gemacht.

Die Wirkstoffzubereitung in Form des Assoziationskomplexes kann zusätzlich zu den oben genannten Komponenten auch einen Extrakt von Acerolafrüchten (Malpighia punidifolia) enthalten. Acerola ist ein kleiner in Westindien, im nördlichen Südamerika, in Mittelamerika, Florida und Texas beheimateter Baum und reich an Vitamin C und anderen Wirkstoffen wie Vitamin A, Thiamin, Riboflavin und Niacin, die zusammen mit verschiedenen anderen Bestandteilen, wie Phosphor, Eisen, Calcium eine komplexe Wirkung entfalten können. Der wäßrige Acerola-Extrakt liegt normalerweise als pulverisiertes Produkt vor.

Als weiterer Wirkstoff in der Gesamtzusammensetzung der kosmetischen Zubereitung und zusätzlich zu dem o.g. Wirkstoffkomplex können in einer besonders bevorzugten Ausführungsform enthalten sein ein oder mehrere der folgenden Bestandteile:

(1) Extrakte oder behandelte Extrakte von freie Radikale bindenden oder feuchtigkeitsbindenden Pflanzen, ausgewählt unter Acerolafrüchten (Malpighia punidifolia), Camellia oleifera, Colunsonia canadensis und Hibiscus sabdariffa;

(2) Extrakte oder behandelte Extrakte von freie Radikale bindenden oder feuchtigkeitsbindenden Algen, ausgewählt unter Omegaplankton mit hohem Anteil an Cerebrosid-Stimulantien, Mikro-Algen der Gattung Chlorella und mit Byssus (Muschelseide) assoziierten Makro-Algen der Gattung Ulva als biotechnologische Proteinfraktion und nachfolgender Assoziierung mit Dextrin, wobei das Produkt im Gemisch mit Peptiderivaten vorliegt, die von α-MSH abgeleitet und mit Xanthin assoziiert sind;

(3) natürliche und synthetische Polymere, ausgewählt unter Chitosanglycolat, Kondensationsprodukte von Milchpulver und aktivierten Fettsäuren;

(4) hartmagnetische Einkristalle aus Bariumhexaferrit mit einer Koerzitivfeldstärke von 3000-5000 Oe und einer Korngröße von 50-1200 nm eingelagert in oder im Gemisch mit asymmetrischen lamellaren Aggregaten aus Phospholipiden und Fluorcarbonen; sowie

(5) weitere Wirk- und Trägerstoffe, ausgewählt unter Hyaluronsäure, Omega CH Aktivator, Behentrimoniumchlorid, Passionsblumenöl sowie modifizierter Kaolin.

Der genannte modifizierte Kaolin wird gemäß WO96/17588 enthalten und ist mit sphärischen $TiO_2$- oder $SiO_2$-Teilchen mit einer Teilchengröße <5 μm modifiziert, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit. Der modifizierte Kaolin kann einen Anteil von 0,1 bis 6 Gew-% haben, bezogen auf die Gesamtmenge des Kosmetikums.

Die genannten hartmagnetischen Teilchen zur Förderung der Durchblutung können solche sein, wie sie in der WO95/03061 beschrieben sind oder solche mit kleineren Teilchengrößen und im Gemisch mit asymmetrischen lamellaren Aggregaten, die bis zum Sättigungsdruck mit Sauerstoff beladen sind, wobei der Anteil der Magnetteilchen, bezogen auf die Gesamtzusammensetzung des Kosmetikums im Bereich von 0,01 bis 10 Gew-% liegen kann.

Die genannten asymmetrischen lamellaren Aggregate sind aus WO94/00098 bekannt und bestehen aus Phospholipiden und mit Sauerstoff beladenem Fluorcarbon oder Fluorcarbongemisch. Der Gehalt an Fluorcarbon liegt im Bereich von 0,2 bis 100 % Gewicht/Volumen, wobei das Phospholipid einen Phosphatidylcholingehalt von mehr als 30 bis 99 Gewichts-% hat, und wobei diese Aggregate eine Hautpenetrierung in Abhängigkeit von der kritischen Löslichkeitstemperatur der Fluorcarbone besitzen.

Die Aggregate können auch zusätzlich allein nur mit Sauerstoff beladen in der kosmetischen Zubereitung vorliegen. Der Anteil kann zwischen 2,5 und 20 Gew-% betragen, bezogen auf die Gesamtzusammensetzung des Kosmetikums.

Diese Aggregate sind Sauerstoffträger und ermöglichen ein Penetrieren des Sauerstoffs in die Haut und damit eine bessere Versorgung der Haut mit Sauerstoff.

Das erfindungsgemäße Präparat enthält weiterhin kosmetische Hilfs- und Trägerstoffe, wie sie üblicherweise in solchen Zubereitungen verwendet werden, z.B. Wasser, Glycerin, Propylenglycol, Konservierungsmittel, Farbstoffe, Pigmente mit färbender Wirkung, Verdickungsmittel, weichmachende Substanzen, feuchthaltende Substanzen, Duftstoffe, Alkohole, Polyole, Elektrolyte, polare und unpolare Öle, Polymere, Copolymere, Emulgatoren, Wachse, Stabilisatoren, gefärbte Pflanzenextrakte, wie fettlöslicher Gardenienextrakt, fettlöslicher Karottenextrakt, Paprika-LS-Extrakt, β-Caroten, Lithospermum-Extrakt sowie aktive Deodorantien.

Es ist weiterhin vorteilhaft, den erfindungsgemäßen Zusammensetzungen entsprechende wasser- und/oder öllösliche UVAoder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl) ester, Benzophenon-Derivate wie 2-Hydroxy-4-methoxybenzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Bevorzugt als Sonnenschutzfilter sind anorganische Pigmente auf Basis von Metalloxiden, wie $TiO_2$, $SiO_2$, ZnO, $Fe_2O_3$, $ZrO_2$, MnO, $Al_2O_3$, die auch im Gemisch untereinander oder mit organischen Filtern eingesetzt werden können.

Besonders bevorzugt als anorganische Pigmente sind agglomerierte Substrate von $TiO_2$ und/oder ZnO, die einen Gehalt an sphärischen und porösen $SiO_2$-Teilchen aufweisen, wobei die $SiO_2$-Teilchen eine Teilchengröße im Bereich von 0,05 μm bis 1,5 μm haben, und neben den $SiO_2$-Teilchen andere anorganische teilchenförmige Stoffe mit sphärischer Struktur vorliegen, wobei die sphärischen $SiO_2$-Teilchen mit den anderen anorganischen Stoffen definierte Agglomerate mit einer Teilchengröße im Bereich von 0,06 μm bis 5 μm bilden.

Besonders vorteilhaft einzusetzende SiO$_2$-Teilchen sind hochmonodisperse, unporöse, sphärische SiO$_2$-Teilchen gemäß DE 3616133, die durch hydrolytische Polykondensation von Tetraalkoxysilan in wäßrig-alkoholisch-ammoniakalischen Medium erzeugt werden, wobei ein Sol von Primärteilchen erzeugt wird und anschließend durch ein kontinuierliches, nach Maßgabe des Abreagierens kontrolliertes Zudosieren von Tetraalkoxysilan die erhaltenen SiO$_2$-Teilchen auf die gewünschte Teilchengröße von etwa 0,05 bis 10 μm bringt.

Pigmente, Pigmentgemische oder Pulver mit pigmentartiger Wirkung, worunter auch solche mit Perlglanz-Effekt zu verstehen sind, können zum Beispiel weiterhin umfassen Glimmer, Kaolin, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, verkapselte und unverkapselte Getreidestärken sowie Glimmer-Titanoxid-organischer Farbstoff.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche Öle wie Maisöl, Baumwollsamenöl, Olivenöl, mineralische Öle, Butylmyristat, Palmitinsäure usw.

Kosmetische Zusammensetzungen mit der erfindungsgemäßen Wirkstoffzubereitung können als O/W- oder W/O-Emulsionen vorliegen. Geeignete Emulgatoren für O/W-Emulsionen sind beispielsweise Anlagerungsprodukte von 2-30 Mol Ethylenoxid an lineare C$_8$-C$_{22}$-Fettalkohole, an C$_{12}$-C$_{22}$-Fettsäuren und an C$_8$-C$_{15}$-Alkylphenole; C$_{12}$-C$_{22}$-Fettsäuremono- und -diester von Anlagerungsprodukten von 1-30 Mol Ethylenoxid an Glycerin; Glycerinmono- und -diester sowie Sorbitanmono- und diester von C$_6$-C$_{22}$-Fettsäuren; Polyol- und Polyglycerinester; Anlagerungsprodukte von Ethylenoxid an Ricinusöl; Betaine wie Kokosalkyldimethylammoniumglycinat oder Kokosacylaminoethylhydroxyethylcarboxymethylglycinat (CTFA: Cocamidopropyl Betaine) sowie ampholytische Tenside.

Geeignete Emulgatoren für W/O-Emulsionen sind beispielsweise Anlagerungsprodukte von 2-15 Mol Ethylenoxid an Ricinusöl; Ester von C$_{12}$-C$_{22}$-Fettsäuren und Glycerin, Polyglycerin, Pentaerythrit, Zuckeralkoholen (z.B. Sorbit), Polyglucosiden (z.B. Cellulose); Polyalkylenglycole; Wollwachsalkohole; Copolymere von Polysiloxan-Polyalkylpolyether.

Der Wassergehalt einer Zubereitung mit dem Wirkstoffkomplex kann in weiten Bereichen schwanken und liegt vorzugsweise bei 5 bis 90 Gew-%, wobei geringe Wassergehalte von ca. 0,5-8 Gew-% insbesondere bei Lippenstiften zu verzeichnen sind.

Die besonders vorteilhafte kosmetische Zubereitung mit der Wirkstoffkomponente (f) schützt in besonders wirksamer Weise gegen den Angriff freier Radikale auf die Haut sowohl allein als auch in Kombination mit anderen Wirkstoffen, und sie zeigt eine überraschende Wirkung auf die Öffnung der Hautporen ähnlich der Wirkungsweise eines Reinigungsmittels (peeling). Dadurch kommen andere Eigenschaften durch weitere Inhaltsstoffe der kosmetischen Zubereitung, wie anhaltende verbesserte Feuchthaltung und Glättung der Haut und somit eine noch mehr verstärkte, langanhaltende Verbesserung des Gesamtzustandes der Haut zur Geltung.

Weiterhin überraschend war es, daß das sowohl in gekapselter als auch ungekapselter Form vorliegende Retinylpalmitat zur gleichen Zeit in den oberen und in den tieferen Hautschichten wirksam wird, und diese Wirksamkeit stabil über einen längeren Zeitraum anhält und die Reparaturwirkung des Assoziationskomplexes verstärkt. Letzteres scheint auf die gleichzeitige Gegenwart von Tocopherylacetat zurückzuführen sein, da nur in dieser Kombination die gleichzeitige und anhaltende Wirkung zu beobachten war.

Die erfindungsgemäße kosmetische Wirkstoffzubereitung schützt in besonders wirksamer Weise gegen den Angriff freier Radikale auf die Haut sowohl allein als auch in Kombination mit anderen Wirkstoffen. Sie hat einen hohen Radikalschutzfaktor zwischen 100 und 3500 x 10$^{14}$ Radikale/mg.

Der Radikalschutzfaktor (RPF) bestimmt die Aktivität einer Substanz zur Bindung freier Radikale gegenüber einer Testsubstanz. Diese Testsubstanz besteht aus einem sehr reaktionsfähigen, halbstabilen Radikal, das mit allen bekannten Antioxidationsmitteln reagiert. Zu solchen Radikalen gehören Nitroxide, wie Proxo (2,2,5,5-Tetramethyl-1-dihydropyrrolinoxy-nitroxid), Tempol (2,2,6,6-Tetramethyl-1-piperidinoxy-4-ol-nitroxid), DTBN (Di-tert-butyl-nitroxid oder vorzugsweise DPPH (1,1-Diphenyl-2-picryl-hydrazyl).

Die Messung des RPF erfolgt in der Weise, daß die Signalamplitude des Testradikals durch Elektronenspinresonanz (ESR/EPR) vor und nach dem Vermischen mit einem Antioxidationsmittel gemessen und daraus der RPF berechnet wird. Für eine Reihe von Standard-Antioxidationsmitteln ist der RPF bekannt, so liegt er für all-trans-Retinol bei 827, all-trans-Retinolacetat bei 196; für DL-α-Tocopherol bei 41200 und für α-Tocopherolacetat bei 48, jeweils x 10$^{14}$ Radikale/mg.

Die erfindungsgemäße kosmetische Wirkstoffzubereitung, die, wenn sie als "Assoziationskomplex" der Bestandteile (a) bis (e) vorliegt, hat bei einer Konzentration von (a) und (b) von jeweils 10 Gew-% allein einen RPF von 1255 und liegt damit sehr hoch gegenüber üblichen Wirkstoffen in Kosmetikformulierungen mit deklarierten Radikalfängern, die Werte von etwa 4 bis 40 erreichen. Dabei sind die eigentlichen Wirkstoffe in (a) und (b) nur in einer Konzentration von maximal 2 Gew-% vorhanden.

Unter "hohem Radikalschutzfaktor" wird in der vorliegenden Erfindung ein Wert von 100 oder höher verstanden, vorzugsweise 1000 oder höher. Dieser Wert kann bei bestimmten erfindungsgemäßen Kombinationen von Pflanzenextrakten und dem eigentlichen Assoziationskomplex auf 10000 und höher gesteigert werden. Entsprechende kosmetische Kompositionen mit solchen Zubereitungen haben Radikalschutzfaktoren je nach Anteil der Zubereitung von z.B. 40 bis 200 oder höher.

Das genaue Meßverfahren für den Radikalschutzfaktor ist beschrieben von Herrling, Groth, Fuchs und Zastrow in Conference Materials "Modern Challenges To The Cosmetic Formulation" 5.5.-7-5.97, Düsseldorf, S. 150-155, Verlag f. chem. Ind. 1997. Dabei wird, ausgehend von der bekannten Konzentration der Testsubstanz (hier: DPPH) oder der Anzahl von dessen freien Radikalen (Radikale pro ml) eine Signalamplitude $S_1$ mittels eines ESR-Spektrometers gemessen. Das Testradikal ist ebenso wie das Antioxidationsmittel in einer (z.B. 0,1 m) Wasser/Alkohollösung gelöst. Dann wird die Signalamplitude $S_2$ des Antioxidationsmittels gemessen. Die normalisierte Differenz zwischen den beiden Signalamplituden ist der Reduktionsfaktor RF.

$$RF = (S_1 - S_2) / S_1$$

Das Ergebnis der Radikalreduzierung der Testsubstanz RC x RF wird zu der Menge der Produkteingabe PI (mg/ml) normalisiert. Dabei ist RC die Menge der Testsubstanz, d.h. die bekannte Anzahl der Radikale der Testsubstanz. Der Radikalschutzfaktor wird nach der folgenden Gleichung berechnet

$$RPF = \frac{RC\ [Radikale/ml]\ x\ RF}{PI\ [mg/ml]}$$

Das Ergebnis ist

$$RPF = N\ x\ 10^{14}\ [Radikale\ pro\ mg],$$

wobei N eine positive reale Zahl ist, und der RPF vereinfacht auf den Zahlenwert von N verkürzt werden kann. Diese Verkürzung ist in den Beispielen der vorliegenden Erfindung benutzt.

Der Radikalschutzfaktor kann mittels eines handlichen und sehr einfach konstruierten ESR-Spektrometers (GALENUS GmbH, Berlin, Deutschland) bestimmt werden und ist eine neue Größe zur Kennzeichnung kosmetischer Produkte hinsichtlich ihrer Fähigkeit, freie Radikale zu binden. Das Verfahren ist ein invitro-Verfahren, bei dem keine individuellen Eigenschaften des kosmetischen Anwenders die Antioxidantien beeinflussen.

Weitere vorteilhafte Wirkungen von Produkten mit der erfindungsgemäßen Wirkstoffzubereitung zusammen mit anderen Wirk- und Trägerstoffen sind eine langanhaltende Verbesserung des Gesamtzustandes der Haut, Verzögerung des Alterungsprozesses der Haut, anhaltende verbesserte Feuchthaltung und Glättung der Haut. Die oben beschriebene besonders vorteilhafte Ausführungsform mit einem zusätzlichen Algen-Peptid-Produkt und hartmagnetischen Bariumhexaferrit-Einkristallen zeigt ein besonders Allergie-reduziertes Risiko nach Allergie- und dermatologischer Testung.

Die Verwendung des erfindungsgemäßen kosmetischen Präparates kann z.B. erfolgen in Sonnencremes, Sonnengelen, Aftersun-Produkten, Tagescremes, Nachtcremes, Masken, Körperlotionen, Reinigungsmilch, Make up's, Lippenstiften, Augenkosmetik, Haarmasken, Haarspülungen, Haarshampoos, Duschgelen, Duschölen, Badeölen und weiteren üblichen Produkten. Besonders vorteilhaft liegt die erfindungsgemäße Wirkstoffzubereitung mit der wahlweisen Komponente (f) in einer Creme, Lotion, einem Make-up, Fluid, Gel oder Lippenstift vor. Zu vorteilhaften kosmetischen Präparaten gehören auch Zahnpasten und Mundwässer unter dem speziellen Aspekt einer Neutralisierung freier Radikale im Mund von Rauchern, sowie als Spezialcreme für Hände und Gesicht von Rauchern. Die Herstellung derartiger Produkte erfolgt auf eine Weise, wie sie dem Fachmann auf diesem Gebiet bekannt ist. Es können bei Wahl entspechender Trägerstoffe auch entsprechende Pharma-Zubereitungen hergestellt werden.

Die Erfindung soll nachstehend durch Beispiele näher erläutert werden. Alle Angaben erfolgen in Gewichtsprozent, sofern nichts anderes angegeben ist.

Herstellung des Wirkstoffkomplexes

Zur Herstellung der Gelgrundlage wurde Gelpulver, wie Carbomer, in Wasser gegeben, darin homogenisiert und anschließend z.B. mit Triethanolamin neutralisiert. Danach erfolgte eine Zugabe von Ethanol und Glycerin zur besseren Einarbeitbarkeit, wobei die entstehende Mischung gut verrührt wurde.

Zu dieser Gelgrundlage wurde ein Gemisch von Phospholipiden (Phoslipon®), Quebracho-Extrakt und Seidenraupenextrakt gegeben und bei einer Temperatur von höchstens 45 °C damit vermischt. Anschließend erfolgte die Zugabe eines weiteren Teiles des obigen Gels oder eines zweiten Gels, wie Guar Propyltriammoniumchlorid, das gut mit dem Gesamtgemisch bei erhöhter Temperatur jedoch unter 45 °C verrührt wurde. Man erhielt auf diese Weise die erfindungsgemäße Wirkstoffzubereitung, im folgenden als "Komplex" bezeichnet.

Für den Fall, wo die Wirkstoffzubereitung weitere Bestandteile, wie Hefeaufschlußprodukt, Acerola-Extrakt oder Extrakte von Tee, Kaffee, Kiwi, Citrus, Kirsche, Papaya oder Hautbaum enthielt, wurde dieser Extrakt der Phospholipidmischung zugesetzt und dann mit dem Gel vermischt.

## Beispiel 1 **Tagescreme**

> **Phase A :** Carbomer 0,2; Glycerin 2,0; Propylenglycol 1,0; dest. Wasser q.s. ad 100;
> **Phase B :** $C_{12}$-$C_{15}$-Alkyl Cetylalkohol 3,7; Stearath 0,5; Jojobaöl 1,0;
> **Phase C :** Triethanolamin 0,2;
> **Phase D :** Wirkstoffkomplex mit (a)-(f) 3,5; Parfüm 0,5; Konservierungsmittel 0,3.

Die Phasen A und B wurden unter Rühren auf 65 ±2 C° erwärmt und die Phase B in der Phase A homogenisiert. Danach erfolgte die Zugabe der Phase C und entsprechendes Homogenisieren. Dann wurde unter Rühren auf etwa 35 °C abgekühlt und die Phase D unter sorgfältigem Vermischen zugesetzt. Der Wirkstoffkomplex enthielt 1,0 % aus Bäckerhefe und nach dem Ultraschallverfahren der DE 4241154C1 gewonnenem SOD-haltigem Enmzym/Vitaminprodukt.

Der zugesetzte Wirkstoffkomplex enthielt 1% trockenes Gel, 7% Phospholipide, 2% Quebracho-Extrakt, 1% Seidenraupen-Extrakt, 1 % SOD aus Hefeaufschlußprodukt. Der Radikalschutzfaktor des Wirkstoffkomplexes betrug 1925, und in der Formulierung lag der RPF bei 49.

## Beispiel 2 **Spezial-Creme**

> **Phase A :** Glycerin 3,0; dest. Wasser q.s. ad 100;
> **Phase B :** Vaseline 22,5; Jojobaöl 5,0;
> **Phase C :** Wirkstoffkomplex mit (a)-(f) 5,5;

Asymmetrische lamellare Aggregate (AOCS) gemäß Beispiel 2 von WO94/00109 10,5; AOCS mit Ba-hexaferrit-Einkristallen gemäß Beispiel 1 von WO95/03061 2,0; Modifizierter Kaolin gemäß Beispiel 1 von WO96/17588 0,3.

Die Phasen A und B wurden unter Rühren auf 65 ±2 C° erwärmt und die Phase B in der Phase A homogenisiert. Danach wurde unter Rühren auf etwa 35 °C abgekühlt und die Phase C unter sorgfältigem Vermischen zugesetzt.

Der Wirkstoffkomplex enthielt 1,0 % aus Weinhefe und nach dem Ultraschallverfahren der DE 4241154C1 gewonnenem SOD-haltigem Enmzym/Vitaminprodukt sowie zusätzlich 0,5 % Vitamin E und 0,5 % Vitamin C. Als Grundbestandteile des Wirkstoffkomplexes waren enthalten 0,5% trockenes Gel, 10% Phospholipide, 1% Quebracho-Extrakt, 2% Seidenraupen-Extrakt. Der Radikalschutzfaktor des Wirkstoffkomplexes betrug 2120, und in der Formulierung lag der RPF bei 41,5.

## Beispiel 3 **Sonnengel**

> **Phase A:** Carbomer 1,5; Glycerin 3,0; Propylenglycol 2,5; dest. Wasser q.s. ad 100;
> **Phase B :** Triethanolamin 1,5;
> **Phase C :** Peptidpräparat MAP-X® gemäß PCT/DE97/02941 1,0; ZnO 2,0; $TiO_2$ 5,0; $SiO_2$ 1,0; Schellack (20 %ige wäßrige Lösung) 1,0;
> **Phase D** : Wirkstoffkomplex mit (a)-(f) 3,5;
> **Phase E :** Parfüm 0,5; Konservierungsmittel 0,3.

Die Phase A wurde unter Rühren auf 60 ±2 C° erwärmt und homogenisiert und auf 45 °C abgekühlt. Die Phase B wurde in der Phase A homogenisiert. Nach dem Abkühlen auf etwa 40 °C erfolgte die Zugabe der Phase C und gutes Homogenisieren. Dann wurde unter Rühren auf etwa 35 °C abgekühlt und die Phasen D und E unter sorgfältigem Vermischen und weiterem Rühren zugesetzt.

Der Wirkstoffkomplex enthielt 1,0 % aus Bierhefe und nach dem Ultraschallverfahren der DE 4241154C1 gewonnenem SOD-haltigem Enmzym/Vitaminprodukt sowie zusätzlich jeweils 0,5 % Vitamin E, Vitamin C und Vitamin A. Als Grundbestandteile des Wirkstoffkomplexes waren enthalten 0,15% trockenes Gel, 20% Phospholipide, 5% Quebracho-Extrakt, 3% Seidenraupen-Extrakt. Der Radikalschutzfaktor des Wirkstoffkomplexes betrug 3050.

## Beispiel 4 **Tages-Creme**

Es wurde eine Zusammensetzung gemäß Beispiel 1 hergestellt, wobei der Wirkstoffkomplex anstelle von 1% SOD aus Hefeaufschluß 20% Tee-Extrakt von schwarzem Tee enthielt. Der Radikalschutzfaktor des Wirkstoffkomplexes betrug 3100.

## Beispiel 5 **Sonnen-Creme**

Es wurde eine Zusammensetzung gemäß Beispiel 1 hergestellt, wobei in Phase A zusätzlich 3% $TiO_2$ und 7,5% Benzophenone-3 enthalten waren. Der Wirkstoffkomplex enthielt anstelle von 1% SOD aus Hefeaufschluß 5% Kaffebohnen-Extrakt von gerösteten Kaffeebohnen und 2% Kiwi-Extrakt. Der Radikalschutzfaktor des Wirkstoffkomplexes betrug 3200.

## Beispiel 6 **Tages-Creme**

Es wurde eine Zusammensetzung gemäß Beispiel 1 hergestellt, wobei zusätzlich in Phase A 1% $TiO_2$ und 0,5 % ZnO enthalten waren. Anstelle von 1% SOD aus Hefeaufschluß enthielt der Wirkstoffkomplex 1% Tee-Extrakt von grünem Tee, 2% Kaffeebohnen-Extrakt von grünen Kaffeebohnen, 1 % Vitamin C und 1% Vitamin E (Tocopherolacetat). Der Radikalschutzfaktor des Wirkstoffkomplexes betrug 5600.

Beispiel 7 **Vergleichsbeispiel**

Es wurden die folgenden Bestandteile eines Wirkstoffkomplexes miteinander vermischt:

> 0,15 % Guar Propyltriammoniumchlorid (Gel); 20 % Phospholipide; 0,1 % Triethanolamin; 1,0 % Vitamin E; 0,1 % Vitamin C; 78,65 % Wasser.

Der gemessene Radikalschutzfaktor der Gesamtzusammensetzung betrug 2.

Beispiel 8 **Fluid auf Emulsionsbasis mit erhöhtem Vitamin A-Gehalt (Vitamin A²)**

> **Phase A :** Carbomer 0,05; Glycerin 2,5; Propylenglycol 0,5; dest. Wasser q.s. ad 100;
> **Phase B :** $C_{12}$-$C_{15}$-Alkyl Cetylalkohol 1,5; Stearath 0,1; Olivenöl 1,0;
> **Phase C :** Triethanolamin 0,05;
> **Phase D :** Komplex mit (a) bis (d) mit 2 % Quebraco-Extrakt, 2% Seidenraupenextrakt, 0,1 % Carbomer, 0,1 % TEA, Wasser = 0,9; sowie (h) Retinylpalmitat und Tocopherylacetat (1:1) als Liposome mit Phospholipiden und mit 0,1 % Micrococcus luteus-Extrakt = 0,1 ; Retinylpalmitat (frei) 0,5;
> **Phase E :** Parfümöl 0,2; Konservierungsmittel 0,3.

Die Phasen A und B wurden unter Rühren auf 65 ±2 C° erwärmt und die Phase B in der Phase A homogenisiert. Danach erfolgte bei etwa 50 °C die Zugabe der Phase C und entsprechendes Homogenisieren. Dann wurde unter Rühren auf etwa 30 °C abgekühlt und die Phase D und die Phase E unter sorgfältigem Vermischen zugesetzt und homogenisiert.

Der Radikalschutzfaktor (RPF) des Komplexes betrug 2100, und der RPF des Fluids lag bei 20.

Beispiel 9 **O/W Antirauch-Tagescreme**

> **Phase A :** Sorbitan monostearate 4; Avocado Oil 3; Oleyl oleate 8;
> **Phase B :** Wasser q.s. ad 100; Propylenglycol 2; Glycerin 5; Carbomer 0,2;
> **Phase B1 :** NaOH 0,4;
> **Phase C :** Konservierungsmittel 0,4;
> **Phase D :** Wirkstoffkomplex mit (a)-(f) mit Vitamin A,E,C,B 5;
> **Phase E :** Parfümöl 0,5

Die Phasen A und B wurden separat bei 80 °C unter intensivem Rühren hergestellt, danach zusammengeführt, gerührt und homogenisiert. Nach Abkühlen auf 60 °C wurde Phase B1 zugesetzt zur Neutralisierung. Nach Abkühlen auf 50 °C erfolgte die Zugabe von Phase C. Die Phasen D und E wurden nacheinander bei 30 °C dem Gemisch zugegeben und homogen verrührt; RPF=39.

Beispiel 10 **Antirauch-Nachtcreme**

Die Creme ist für Raucherhaut mit Reparatureffekt und dient zugleich der Vorbeugung für den Tag.

> **Phase A :** Vaseline 8,5; Jojoba Oil 3,0; Stearinsäure 3,8;
> **Phase B :** Wasser q.s. ad 100; Glycerin 5; Carbomer ;0,3
> **Phase C :** Triethanolamin 0,3;
> **Phase D :** Konservierungsmittel 0,4;
> **Phase E :** Wirkstoffkomplex mit (a)-(f) mit Vitamin A, E, C, B und 2 % Aloe vera 10,0 ; Parfümöl 0,1.

Die Arbeitsweise entsprach der von Beispiel 9; RPF=48.

Beispiel 11 **Handcreme gegen braune Raucherfinger**

> **Phase A :** Cetylakohol 8,5; Stearinsäure 3,8;
> **Phase B :** Wasser q.s. ad 100; Glycerin 2; Carbomer 0,9;
> **Phase C :** Triethanolamin 0,3;
> **Phase D :** Vitamin E 1,0; Aloe vera 1,0; Konservierungsmittel 0,4; Wirkstoffkomplex mit (a)-(f) mit Vitamin A, E, C 5,0; Parfümöl 1,4; Whitening Komplex 1,0.

Die Arbeitsweise entsprach der von Beispiel 9. RPF=35.

**Patentansprüche**

1. Kosmetische Wirkstoffzubereitung mit hohem Radikalschutzfaktor, **gekennzeichnet durch** einen Gehalt an

> (a) einem **durch** Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew- % Gallussäure enthält, wobei der Gehalt von (a), das in einer an Mikrokapseln gebundenen Wirkstoffkonzentration von 2 Gew- % vorliegt, im Bereich von 0,1 bis 10 Gew- % liegt;

(b) einem **durch** Extraktion gewonnenen Seidenraupenextrakt, der das Peptid Cecropine, Aminosäuren und ein Vitamingemisch enthält, wobei der Gehalt von (b) im Bereich von 0,1 bis 10 Gew-% liegt;

(c) einem nichtionischen, kationischen oder anionischen Hydro-Gel oder Gemisch von Hydro-Gelen mit einem Gehalt von (c) im Bereich von 0,1 bis 5 Gew-%;

(d) einem oder mehreren Phospholipiden mit einem Anteil von 0,1 bis 30 Gew-%;

(e) Wasser.

2. Zubereitung nach Anspruch 1, **dadurch gekennzeichnet, daß** sie zusätzlich enthält

(f) einem wenigstens 150 Einheiten Superoxiddismutase pro ml enthaltenden Ultraschall-Aufschlußprodukt einer Hefe, wobei der Gehalt des Aufschlußproduktes (f) im Bereich von 0 bis 4 Gew-% liegt; und

(g) einem Extrakt von Acerolafrüchten Malpighia punidifolia, wobei der Gehalt (g) im Bereich von 0 bis 30 Gew-% liegt; jeweils bezogen auf das Gesamtgewicht der Wirkstoffzubereitung.

3. Zubereitung nach Anspruch 1, **dadurch gekennzeichnet, daß** sie zusätzlich enthält

(h) ein Gemisch aus 0,1 Gew-% Micrococcus luteus-Extrakt, Retinylpalmitat und Tocopherylacetat in mit Phospholipiden zubereiteter liposomaler Form, und zusätzlich freies Retinylpalmitat, und wobei die Bestandteile der Zubereitung, bezogen auf das Gesamtgewicht eines kosmetischen Präparates, wie folgt vorhanden sind: Wirkstoffkapseln gemäß (a) und (b) im Bereich von 0,1 bis 10 Gew-%, Hydro-Gel gemäß (c) im Bereich von 0,1 bis 5 Gew-%, gekapseltes Retinylpalmitat gemäß (h) 0,001 bis 5 Gew-%, gekapseltes Tocopherylacetat gemäß (h) 0,001 bis 2 Gew-%, freies Retinylpalmitat gemäß (h) 0,1 bis 5 Gew-%, Phospholipide 0,2 bis 5 Gew-%.

4. Zubereitung nach Anspruch 3, **dadurch gekennzeichnet, daß** der Anteil der Bestandteile in folgenden Bereichen liegt: Wirkstoffkapseln gemäß (a) und (b) im Bereich von 0,5 bis 3 Gew-%, Hydro-Gel gemäß (c) im Bereich von 0,1 bis 3 Gew-% gekapseltes Retinylpalmitat gemäß (h) 0,05 bis 2 Gew-% gekapseltes Tocopherylacetat gemäß (h) 0,05 bis 1 Gew-% freies Retinylpalmitat gemäß (h) 0,5 bis 2 Gew-%.

5. Zubereitung nach Anspruch 1, **gekennzeichnet durch** einen Radikalschutzfaktor im Bereich von 100 bis 3500 Radikale pro mg, gemessen **durch** Bestimmung der Anzahl freier Radikale einer Lösung einer Testsubstanz ($S_1$) mittels Elektronenspinresonanz (ESR) im Vergleich mit dem ESR-Meßergebnis der kosmetischen Wirkstoffzubereitung nach der Beziehung RPF = (RC x RF) / PI worin RF = ($S_1$-$S_2$) / $S_1$ ; RC = Konzentration der Testsubstanz (Radikale/ml); PI = Konzentration der Wirkstoffzubereitung (mg/ml).

6. Zubereitung nach Anspruch 1, **dadurch gekennzeichnet, daß** der Extrakt (a) wenigstens 99 Gew-% Proanthocyanidin-Oligomere und höchstens 1 Gew-% Gallussäure enthält.

7. Zubereitung nach Anspruch 1, **dadurch gekennzeichnet, daß** die in (b) enthaltenen Aminosäuren Aspertinsäure, Asparagin, Threonin, Serin, Glutaminsäure, Prolin, Glycin, Alanin, Valin, Cystein, Methionin, Isoleucin, Leucin, Tyrosin, Phenylalanin, Lysin, Histidin, Arginin umfassen.

8. Zubereitung nach Anspruch 1, **dadurch gekennzeichnet, daß** das in (b) enthaltende Vitamingemisch die Vitamine $B_1$, $B_2$, $B_5$, $B_6$, $B_8$, $B_9$, $B_{12}$, PP, A, E und C umfaßt.

9. Zubereitung nach Anspruch 1, **dadurch gekennzeichnet, daß** die Wirkstoffzubereitung zusätzlich ein Gemisch der Vitamine A, E und C oder die Vitamine einzeln enthält.

10. Zubereitung nach Anspruch 1, **dadurch gekennzeichnet, daß** sie als Assoziationskomplex vorliegt zwischen den Phospholipiden (d), die die Komponenten (a) und (b) teilweise einschließen, und dem Gel (c) mit dem Wasser (e).

11. Zubereitung nach Anspruch 1, **dadurch gekennzeichnet, daß** die Wirkstoffzubereitung in einer kosmetischen Zusammensetzung vorliegt, die weiterhin noch einen oder mehrere der folgenden Bestandteile enthält:

(1) Extrakte oder behandelte Extrakte von freie Radikale bindenden oder feuchtigkeitsbindenden

(1.1) Pflanzen, ausgewählt unter Acerolafrüchten (Malpighia punidifolia), Camellia oleifera, Colunsonia canadensis und Hibiscus sabdariffa, oder

(1.2) Algen, ausgewählt unter Omegaplankton mit hohem Anteil an Cerebrosid-Stimmulantien, Mikro-Algen der Gattung Chlorella und mit Byssus (Muschelseide) assoziierten Makro-Algen der Gattung Ulva als biotechnologische Proteinfraktion und nachfolgender Assoziierung mit Dextrin, wobei das Produkt im Gemisch mit Peptiderivaten vorliegt, die von α-MSH abgeleitet und mit Xanthin assoziiert sind ;

(2) Hefeaufschlußprodukte, ausgewählt unter Bäckerhefe, Brauereihefe und Weinhefe und hergestellt nach einer schonenden Ultraschallbehandlung der wäßrigen Hefen;

(3) natürliche und synthetische Polymere, ausgewählt unter Chitosanglycolat, Kondensationsprodukte von Milchpulver und aktivierten Fettsäuren;

(4) hartmagnetische Einkristalle aus Bariumhexaferrit mit einer Koerzitivfeldstärke von 3000-5000 Oe und einer Korngröße von 50-1200 nm eingelagert in oder im Gemisch mit asymmetrischen lamellaren Aggregaten aus Phospholipiden und Fluorcarbonen; und

(5) weitere Wirkstoffe, ausgewählt unter Chitosanglycolat, Hyaluronsäure, Omega CH Aktivator, Behentrimoniumchlorid, Passionsblumenöl, sowie Trägerstoffe;

(6) Gemischen davon.

**12.** Zubereitung nach Anspruch 1, **dadurch gekennzeichnet, daß** die Bestandteile (a) und (b) in Konzentrationen von jeweils 0,1 bis 3 Gew-% in der Wirkstoffzubereitung vorliegen.

**13.** Zubereitung nach Anspruch 1 oder 11, **dadurch gekennzeichnet, daß** sie zusätzlich einen Gehalt von 0,1 bis 20 Gew-% Pflanzenextrakte aufweist, ausgewählt aus der Gruppe, bestehend aus Citrusschalen- oder -blatt-Extrakte (Citrus bigaradia, Citrus hystrix, Citrus aurantifolia, Citrofurtunella microcarpa, Citrus aurantium, Citrus reticulata), Bitterorange-Extrakt (Schale oder Frucht), Kirsch-Extrakt der spanischen Cherry-Kirsche, Kiwi-Extrakt (Actinidia chinensis), Papayafrucht-Extrakt (Caricae papayae), Tee-Extrakt [Blätter von grünem oder schwarzem Tee, Blätter oder Rinde von New Jersey Tee (Ceanthus velutinas)], Kaffeebohnen-Extrakt (INCI-Name: Coffee Bean Extract; von grünen oder gerösteten Bohnen), Prunus-Extrakt (Prunus armeniaca, Prunus dulcis, Prunus persica, Prunus domestica, Prunus spinosa, Prunus serotina, Prunus virginiana), Extrakte der Rinde des mexikanischen Hautbaumes (Mimosa tenuiflora), Angelikawurzel-Extrakt (Angelica archangelica) und dem restlichen Anteil Trägerstoffe oder weitere Wirkstoffe und Trägerstoffe.

**14.** Verwendung der kosmetischen Wirkstoffzubereitung mit hohem Radikalschutzfaktor nach einem der Ansprüche 1 bis 13 zusammen mit anderen kosmetischen Stoffen, wie weiteren Wirkstoffen, Hilfsstoffen und Trägerstoffen in kosmetischen Zubereitungen wie Cremes, Gelen, Lotionen, Masken, Make-up's, Shampoos, Stiften, ölen, Mascara, entsprechenden Sonnenschutzpräparaten sowie Zahnpasten und Mundwässern.

## Claims

**1.** Cosmetic active substance preparation with a high radical protection factor, which comprises a content of

a) a product obtained by extraction of the bark of Quebracho blanco and subsequent enzymatic hydrolysis, containing at least 90 percent by weight of proanthocyanidine oligomers and up to 10 percent by weight of gallic acid, where the content of (a), which is available in a concentration of 2 percent by weight linked to a microcapsule ranges from 0.1 to 10 wt.%;

(b) an extract of the silkworm obtained by extraction, containing the peptide cecropine, amino acids and a vitamin mix, where the content of (b) ranges from 0.1 to 10 wt.%;

(c) a non-ionic, cationic or anionic hydrogel or mixture of hydrogels, where the content of (c) ranges from 0.1 to 5 wt%;

(d) one or several phospholipids in the range of 0.1 up to 30 wt.%;

(e) water.

**2.** Preparation according to claim 1, **characterised by** additionally comprising

(f) an ultrasound decomposition product of a yeast containing at least 150 units of superoxide dismutase per ml, wherein the content of the decomposition product (f) is in the range from 0 to 4 wt.%, and

(g) an extract of acerola fruits Malpighia punidifolia, wherein the content (g) is in the range from 0 to 30 wt.%; related to the total weight of the active substance preparation each.

**3.** Preparation according to claim 1, **characterised by** additionally comprising

(h) a mixture of 0.1 wt.% of Micrococcus luteus extract, retinyle palmitate and tocopherylacetate in liposomal form prepared with phospholipids, and additionally free retinyle palmitate and wherein the portions of the components contained in the preparation relative to the total weight of a cosmetic preparation are as follows: active substance capsules according to (a) and (b) ranging from 0.1 to 10 wt.%, hydro gel according to (c) ranging from 0.1 to 5 wt.%, encapsulated retinyle palmitate according to (h) ranging from 0.001 to 5 wt.%, encapsulated tocopherylacetate according to (h) 0.001 to 2 wt. %, free retinyle palmitate according to (h) 0.1 to 5 wt.%, phospholipids 0.2 to 5 wt.%.

4. Preparation according to claim 3, **characterised in that** the portions of the components lie within the following ranges: active substance capsules according to (a) and (b) ranging from 0.5 to 3 wt.%, hydro gel according to (c) ranging from 0.1 to 3 wt. %, encapsulated retinyle palmitate according to (h) ranging from 0.05 to 2 wt.%, encapsulated tocopherylacetate according to (h) ranging from 0.05 to 1 wt.%, free retinyle palmitate according to (h) ranging from 0.5 to 2 wt.%.

5. Preparation according to claim 1, **characterised in** a radical protection factor in the range from 100 to 3500 radicals per mg, measured by determining the number of free radicals of a solution of a test substance ($S_1$) by electron spin resonance (ESR) as compared with the ESR measurement result of the cosmetic active substance preparation according to the relationship
$RPF = (RC \times RF) / PI$, where $RF = (S_1-S_2) / S_1$ ; RC = concentration of the test substance (radicals per ml); PI = concentration of the active substance preparation (mg per ml).

6. Preparation according to claim 1, **characterised in that** the extract (a) contains at least 99 wt.% of proanthocyanidine oligomers and up to 1 wt.% of gallic acid.

7. Preparation according to claim 1, **characterised in that** the amino acids contained in (b) comprise aspertine acid, asparagine, threonine, serine, glutamic acid, proline, glycine, alanine, valine, cysteine, methionine, isoleucine, leucine, tyrosine, phenylalanine, lysine, histidine, arginine.

8. Preparation according to claim 1, **characterised in that** the vitamin mixture included in (b) comprises the vitamins $B_1$, $B_2$, $B_5$, $B_6$, $B_8$, $B_9$, $B_{12}$, PP, A, E and C.

9. Preparation according to claim 1, **characterised in that** the active substance preparation contains an additional mixture of the vitamins A, E and C or each of these vitamins individually.

10. Preparation according to claim 1, **characterised in that** it exists as an association complex between the phospholipids (d), partially comprising the components (a) and (b) and the gel (c) with the water (e).

11. Preparation according to claim 1, **characterised in that** the active substance preparation exists as cosmetic composition, further comprising one or several of the following components:

(1) extracts or treated extracts binding free radicals or moisture of

(1.1) plants selected among acerola fruits (Malpighia punidifolia), Camellia oleifera, Colunsonia canadensis and Hibiscus sabdariffa; or
(1.2) algae selected among omega plankton, providing a high portion of cerebrosid stimulants, microalgae of the chlorella species and macro algae of the ulva species associated with byssus (mussel silk) as biotechnological protein fraction and subsequently associated with dextrine, wherein the product appears in the mixture with peptide derivates derived from α-MSH and associated with xanthin;

(2) yeast decomposition products selected among
baker's yeast, brewer's yeast, wine yeast and made according to a non-harming ultrasound treatment of the aqueous yeasts;
(3) natural and synthetic polymers selected among chitosanglycolate, condensed products of desiccated milk, and activated fatty acids;
(4) magnetically hard single crystals of bariumhexaferrite having a coercive field intensity of 3000 - 5000 Oe and a grain size of 50-1200 nm intercalated in or mixed with asymmetric lamellar aggregates of phospholipids and fluorocarbons ; and
(5) other active substances selected among chitosanglycolate, hyaluronic acid, omega CH activator, behentrimonium chloride, passion flower oil and carrier substances;
(6) mixtures thereof.

12. Preparation according to claim 1, **characterised in that** the concentration of the components (a) and (b) in the active substance ranges from 0.1 to 3 wt. % each.

13. Preparation according to claims 1 or 11, **characterized in that** it comprises an additional portion of 0.1 to 20 wt.% of plant extracts selected from the group consisting of citrus peel or leaf extracts (Citrus bigaradia, Citrus hystrix, Citrus aurantifolia, Citrofurtunella microcarpa, Citrus aurantium, Citrus reticulata), petitgrain extract (peel or fruit), extract of the Spanish cherry, kiwi extract (Actinidia chinensis), papaya fruit extract (Caricae papayae), tea extract [leaves of green or black tea, leaves or bark of new jersey tea (Ceanthus velutinas)], coffee bean extract (INCI name: coffee bean extract; of green or roasted beans), prunus extract (Prunus armeniaca, Prunus dulcis, Prunus persica, Prunus domestica, Prunus spinosa, Prunus serotina, Prunus virginiana), extracts of the bark of the Mexican skin tree (Mimosa tenuiflora), angelica root extract (Angelica archangelica); and the remaining portion of carrier substances or other active substances and carrier substances.

14. Use of the cosmetic substance preparation with high radical protection factor according to each of claims 1 through 13 in combination with other cosmetic substances such as other active substances, auxiliary substances and carrier substances in cosmetic preparations such as creams, gels, lotions, masks, makeup's, shampoos, sticks, oils, mascara, corresponding sun protection preparations as well as tooth paste and mouthwashes.

**Revendications**

1. Préparation cosmétique d'agents actifs avec facteur élevé de protection contre les radicaux libres, **caractérisée par** une teneur en

(a) un produit obtenu par extraction de l'écorce de *Quebracho blanco* et ensuite, hydrolyse enzymatique, lequel contient au moins 90% en poids d'oligomères de proanthocyanidine et au maximum 10% en poids d'acide gallique, où la teneur en (a), qui est présent en une concentration en agent actif lié à des microcapsules de 2% en poids, se situe dans l'intervalle allant de 0,1 à 10% en poids ;

(b) un extrait de ver à soie obtenu par extraction, qui contient le peptide cecropine, des acides aminés et un mélange de vitamines, où la teneur en (b) se situe dans l'intervalle allant de 0,1 à 10% en poids ;

(c) un hydrogel non ionique, cationique ou anionique ou un mélange d'hydrogels avec une teneur en (c) située dans l'intervalle allant de 0,1 à 5% en poids ;

(d) un ou plusieurs phospholipides avec une proportion de 0,1 à 30% en poids ;

(e) de l'eau.

2. Préparation selon la revendication 1, **caractérisée en ce qu'**elle contient en outre :

(f) un produit de désintégration par ultra-sons d'une levure, contenant au moins 150 unités de superoxyde dismutase par ml, où la teneur en produit de désintégration (f) se situe dans l'intervalle allant de 0 à 4% en poids ;

(g) un extrait du fruit acérola *Malpighia punicifolia*, où la teneur en (g) se situe dans l'intervalle allant de 0 à 30% en poids ;

chaque fois sur base du poids total de la composition d'agent actif.

3. Préparation selon la revendication 1, **caractérisée en ce qu'**elle contient en outre :

(h) un mélange de 0,1% en poids d'extrait de *Micrococcus luteus*, du palmitate de rétinyle et de l'acétate de tocophéryle sous une forme liposomique préparée avec des phospholipides, et en outre, le palmitate de rétinyle libre, et où les constituants de la composition, sur base du poids total d'une préparation cosmétique, sont présents de la manière suivante : capsules d'agent actif selon (a) et (b) dans l'intervalle allant de 0,1 à 10% en poids, hydrogel selon (c) dans l'intervalle de 0,1 à 5% en poids, palmitate de rétinyle encapsulé selon (h) 0,001 à 5% en poids, acétate de tocophéryle encapsulé selon (h) 0,001 à 2% en poids, palmitate de rétinyle libre selon (h) 0,1 à 5% en poids, phospholipides 0,2 à 5% en poids.

4. Préparation selon la revendication 3, **caractérisée en ce que** la proportion des constituants se situe dans les intervalles suivants : capsules d'agent actif selon (a) et (b) dans l'intervalle allant de 0,5 à 3% en poids, hydrogel selon (c) dans l'intervalle de 0,1 à 3% en poids, palmitate de rétinyle encapsulé selon (h) 0,05 à 2% en poids, acétate de tocophéryle encapsulé selon (h) 0,05 à 1% en poids, palmitate de rétinyle libre selon (h) 0,5 à 2% en poids.

5. Préparation selon la revendication 1, **caractérisée par** un facteur de protection contre les radicaux libres situé dans l'intervalle allant de 100 à 3500 radicaux par mg, mesuré par détermination du nombre de radicaux libres d'une solution d'une substance à tester $(S_1)$ à l'aide de la résonance de spin électronique (ESR) par comparaison au résultat de mesure ESR de la préparation cosmétique d'agents actifs selon la relation :

$$RPF = (RC \times RF) / PI$$

où $RF = (S_1-S_2)/S_1$ ; RC = concentration de la substance à tester (radicaux/ml) ; PI = concentration de la préparation d'agents actifs (mg/ml).

6. Préparation selon la revendication 1, **caractérisée en ce que** l'extrait (a) contient au moins 99% en poids d'oligomères de proanthocyanidine et au maximum 1% en poids d'acide gallique.

7. Préparation selon la revendication 1, **caractérisée en ce que** dans (b), les acides aminés présents sont l'acide aspartique, l'asparagine, la thréonine, la sérine, l'acide glutamique, la proline, la glycine, l'alanine, la valine, la cystéine, la méthionine, l'isoleucine, la leucine, la tyrosine, la phénylalanine, l'histidine, l'arginine.

8. Préparation selon la revendication 1, **caractérisée en ce que** dans (b), le mélange de vitamines présent contient les vitamines B1, B2, B5, B6, B8, B9, B12, PP, A, E et C.

9. Préparation selon la revendication 1, **caractérisée en ce que** la préparation d'agents actifs contient en outre, un mélange de vitamines A, E et C ou les vitamines individuellement.

10. Préparation selon la revendication 1, **caractérisée en ce qu'**elle se présente comme un complexe d'association entre les phospholipides (d), qui renferment partiellement les composants (a) et (b), et le gel (c) avec l'eau (e).

11. Préparation selon la revendication 1, **caractérisée en ce que** la préparation d'agents actifs se présente comme une préparation cosmétique, qui contient en outre, encore un
   ou plusieurs des constituants suivants :

   (1) extrait ou extrait traité de

   (1.1) plantes, choisies parmi les fruits acérola (*Malpighia punicifolia*), *Camellia oleifera, Colunsonia canadensis* et *Hibiscus sabdariffa*, ou
   (1.2) algues, choisies parmi l'omégaplancton avec une teneur élevée en stimulants cérébroside, les microalgues des genres Chlorella et les macroalgues associées à des byssus (mollusque bivalve) du genre Ulva, comme fraction protéine biotechnologique et association suivante avec la dextrine, où le produit est présent en mélange avec des dérivés peptidiques, qui dérivent de l'α-MSH et associés à la xanthine,
   se liant aux radicaux libres ou liant l'humidité ;

   (2) des produits de dégradation de levure, choisis parmi la levure de boulanger, la levure de brasserie et la levure vinicole et préparés par un traitement aux ultra-sons des levures aqueuses ;
   (3) des polymères naturels et synthétiques, choisis parmi le glycolate de chitosan, des produits de condensation de poudre de lait et d'acides gras activés ;
   (4) des monocristaux fortement magnétiques en hexaferrite de baryum avec une force de champ coercitif allant de 3000-5000 Oe et une granulométrie allant de 50-1200 nm, piégées dans ou en mélange avec des agrégats lamellaires asymétriques de phospholipides et fluorocarbones, et
(5) d'autres agents actifs, choisis parmi le glycolate de chitosan, l'acide hyaluronique, l'activateur oméga CH, le chlorure de béhéntrimonium, l'huile de fruits de la passion, ainsi que des supports,
(6) leurs mélanges.

12. Préparation selon la revendication 1, **caractérisée en ce que** les constituants (a) et (b) sont présents en des concentrations de chaque fois, 0,1 à 3% en poids dans la préparation d'agents actifs.

13. Préparation selon la revendication 1 ou 11, **caractérisée en ce qu'**elle présente en outre, une teneur de 0,1 à 20% en poids d'extraits de végétaux, choisis parmi le groupe consistant en des extraits de zeste ou de feuille de citron (*Citrus bigaradia, Citrus hystrix, Citrus autantifolia, Citrofurtunella microcarpa, Citrus aurantium, Citrus reticulata*), de l'extrait d'orange amère (zeste ou fruit), de l'extrait de cerises de cerises espagnoles, de l'extrait de kiwi (*Actinidia chinensis*), de l'extrait de papaye (*Caricae papayae*), de l'extrait de thé (feuilles de thé vert ou noir, feuille ou écorce de thé du New jersey (*Ceanthus velutinas*)), de l'extrait de grains de café (nom INCI : Coffee bean extract ; de grains verts ou torréfiés), d'extrait de prunus (*Prunus armeniaca, prunus dulcis, Prunus persica, Prunus domestica, Prunus spinosa, Prunus serotina, Prunus virginiana*), des extraits d'écorce d'arbre à peau du Mexique (*Mimosa tenuiflora*), de l'extrait de racine d'angélique (*Angelica archangelica*), et la proportion résiduelle de supports ou d'autres auxiliaires et supports.

14. Utilisation des préparations cosmétiques de principes actifs avec facteur élevé de protection contre les radicaux libres, selon l'une quelconque des revendications 1 à 13, avec d'autres substances cosmétiques, comme d'autres agents actifs, auxiliaires et supports, dans des préparations cosmétiques comme des crèmes, gels, lotions, masques, maquillages, shampooings, stifs, huiles, mascaras, préparations appropriées de protection solaire ainsi que pâtes dentaires et eaux buccales.